# EUROPEAN PATENT SPECIFICATION

(11) **EP 3 013 375 B1**
(45) Date of publication and mention of the grant of the patent: **27.02.2019**
(21) Application number: 14816701.8
(22) Date of filing: 25.06.2014
(51) Int. Cl.: A61L 9/04, A61L 9/12

(54) **AIR FRESHENER WITH CLIP**
LUFTERFRISCHER MIT CLIP
ASSAINISSEUR D'AIR À ATTACHE

(30) Priority: 25.06.2013 US 201361839138 P
(43) Date of publication of application: 04.05.2016
(73) Proprietor: Scent2Market Inc., Yonkers, NY 10701 (US)
(72) Inventor: D'AMICO, Daniel, M., South Salem, NY 10590 (US)
(74) Representative: Fritzsche, Thomas
(86) International application number: PCT/US2014/044130
(87) International publication number: WO 2014/210180

(56) References cited:
- JP-A- 2005 239 254
- RU-U1- 116 044
- US-A- 2 351 267
- US-A- 2 579 715
- US-A- 5 478 505
- US-A- 5 478 505
- US-A1- 2003 064 009
- US-A1- 2005 169 793

## Description

### BACKGROUND OF THE INVENTION

### Field of the Invention

The invention relates to the fields of scent and aroma management. This may include, for example, but not be limited to increasing the amount of desirable aroma, fragrance, or odor neutralizer in a room, a vehicle, or another area.

### Background of the Related Art

Many devices for providing a fragrance to an area over a period of time are known. Generally, these devices operate by allowing a fragrance contained in the device to passively diffuse from the device and into the atmosphere. In some cases these devices are suspended from a loop, wire, string, or other connector, allowing them to depend from a knob, mirror, or the like.

In some cases distribution of a fragrance is assisted by actively forcing air through the housing of a device. This may be done by including a fan or other air-moving device as an integral part of the fragrance device. It may also be accomplished by securing the fragrance device in close proximity to another source of moving air. One variety of the latter kind of device is shown, for example, in United States Patent No. 8,460,609, which shows a fragrance device in which an automobile vent is used to provide an air flow to assist in the distribution of a fragrance.

JP 2005 239254 A discloses a hanging type container which is self-supporting, and is fitted with a lid which covers a front opening of a container body and has window holes for volatilization at the position corresponds to the opening. The container also includes a hook erected on the top of the container body from which the hook can be folded, and a locking means on which the folded hook is locked.

US 2005/0169793 A1 discloses an air freshener device, which includes a scent material interspersed within a polymer body or a polymer gel, and capable of dispersing out of the polymer gel. The polymer body and a polymer gel can be flexible and compressible, and also resilient and returnable to its original configuration. In addition, the polymer body and the polymer gel can have a freestanding, self-supported, three-dimensional shape.

US 5 478 505 A discloses an air-treating article for dispensing a volatilizable material, such as air freshener fragrance, into the atmosphere of an enclosed area, such as an automobile interior.

The device includes a housing and a reservoir of the volatilizable material therein, and also includes a dual-configured, articulating attachment clip which enables attachment of the device at a high-air-flow station, such as the forced air vent grille of the automobile, or at a low-air-flow station, such as the sun visor of the vehicle. The device enables use of the consistent delivery rates of a low-density polyethylene membranes while at the same producing a higher delivery rate than normally achieved with such membranes.

Unfortunately, known solutions for fragrance distribution may suffer from a number of disadvantages. For example, a consumer may purchase a device meant to depend from a loop, but then decide that the more aggressive fragrancing made available by a secured model is either permanently or temporarily desired. In another situation a consumer using a secured fragrance device may decide that while both the air flow and the fragrance are still desired, the enhanced fragrancing provided by securing the fragrance device to a vent is not still desired. Unfortunately, such a useful device has not previously been proposed.

### BRIEF SUMMARY OF THE INVENTION

It would be helpful to have a fragrance distribution device that retained maximum flexibility in potential location of the device and fragrance delivery. Embodiments of the invention provide a fragrance distribution device comprising a housing, and a fragrance in a polymer or thermoplastic carrier. The housing includes an aperture into which a hook, clip, adhesive peg, or other attaching device may be secured. Typically the aperture is covered by a decal (also referred to as a label) or other cover until its use is desired. In some cases the attaching device is held by a recess in the air freshener when not in use.

The attaching device is removably secured to the housing. By "removably secured" it is meant that the attaching device may be repeatedly removed from and replaced in the housing without damaging the housing or the attaching device. This may be accomplished, for example, but an interference fit.

Other aspects, objectives and advantages of the invention will become more apparent from the following detailed description when taken in conjunction with the accompanying drawings.

### BRIEF DESCRIPTION OF THE DRAWINGS

The accompanying drawings incorporated in and forming a part of the specification illustrate several aspects of the present invention and, together with the description, serve to explain the principles of the invention. In the drawings:

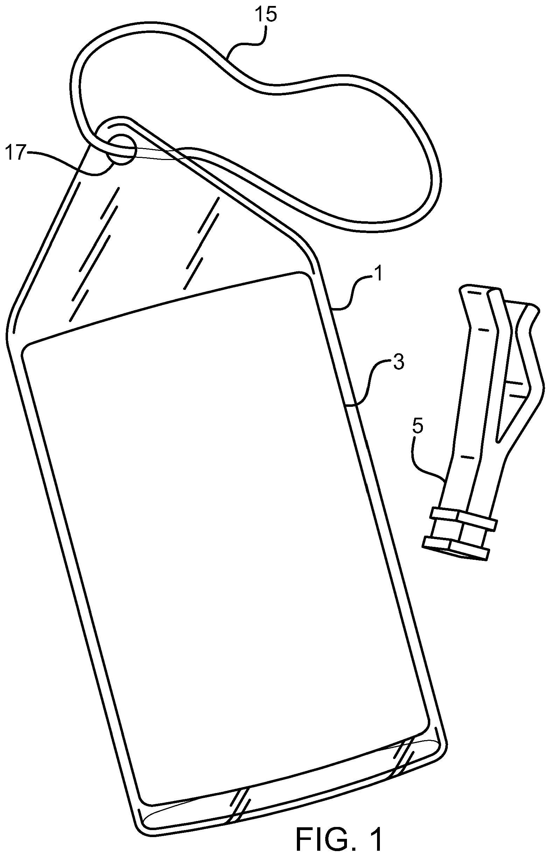

FIG. 1 shows a fragrance device according to one embodiment of the invention, in which a housing **1** includes a label 3. An attaching device, in this case a clip 5 is also shown.

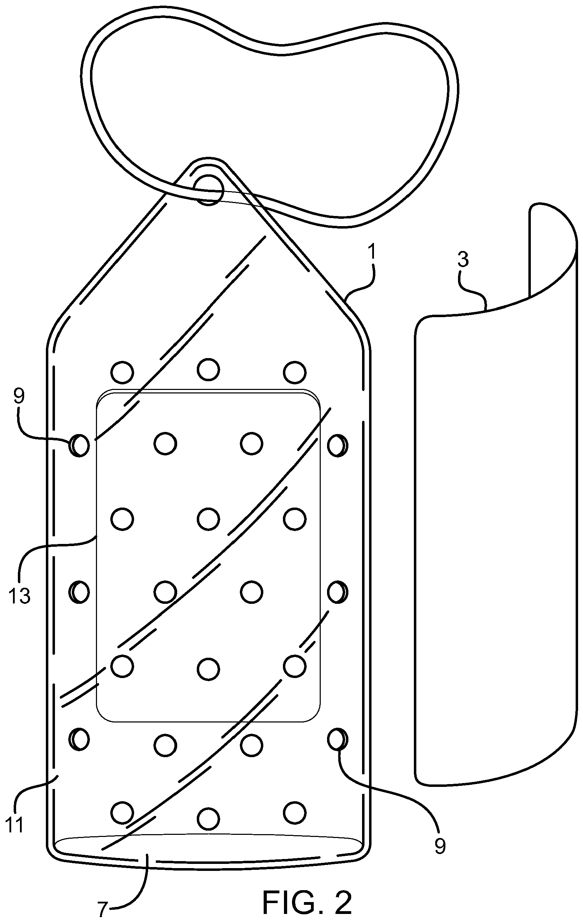

FIG. 2 shows the fragrance device of FIG. 1 with the label 3 removed from the housing **1.** Through the walls of the housing (which is transparent in this embodiment but need not be transparent in all embodiments), the polymer fragrance carrier 7 is visible. A number of circular fragrance vents 9 are shown, and on each side of the housing are additional fragrance vents **11.** Aperture 13 is also included. Also shown is loop **15,** which is secured through the housing by hole **17.**

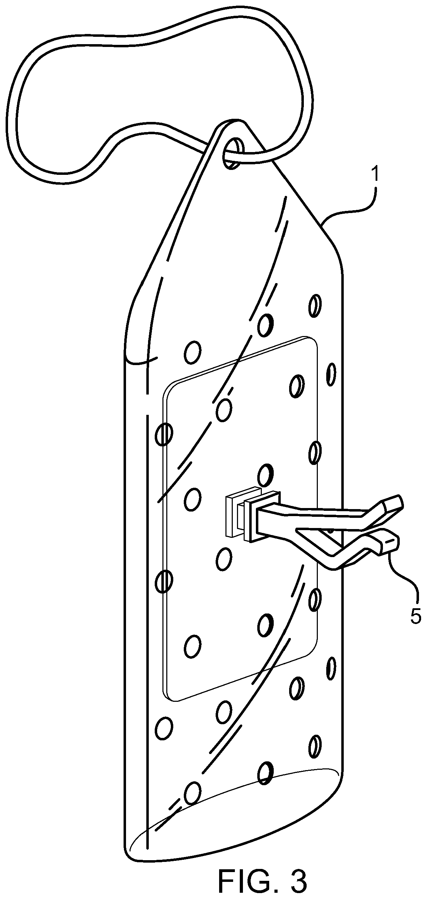

FIG. 3 shows an assembled fragrance device including the housing **1** connected to the clip 5.

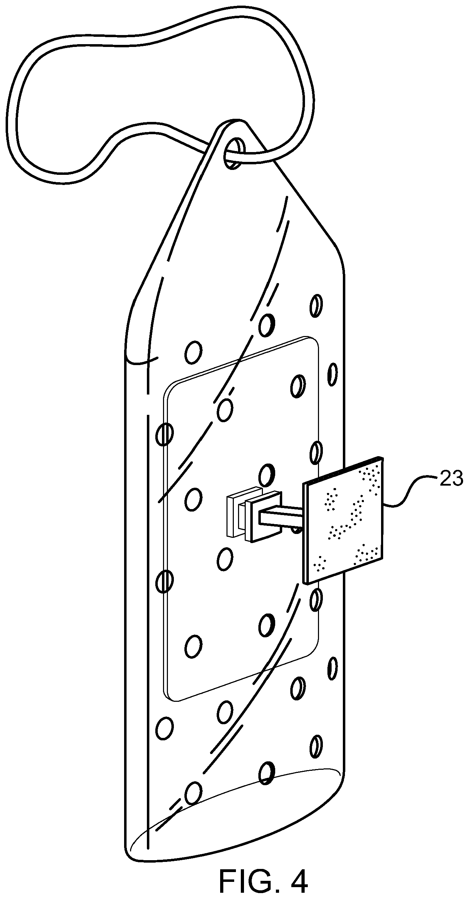

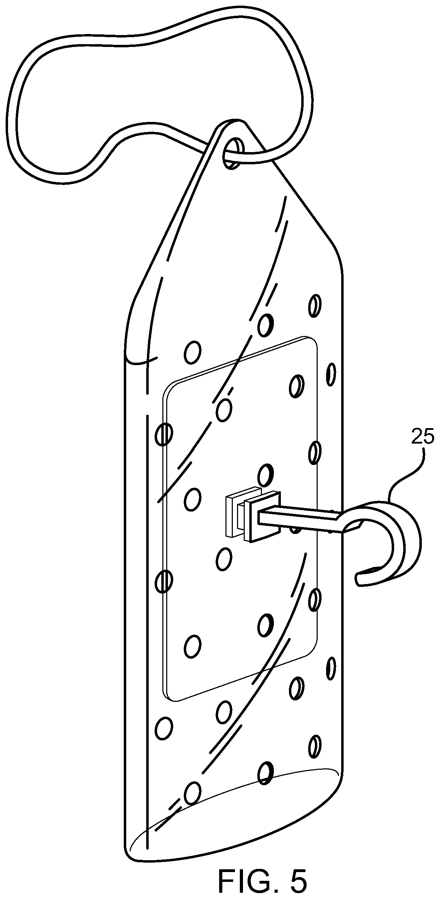

FIG. 4 and FIG. 5 show embodiments of the invention with an adhesive peg 23 and a hook 25, respectively, for attachment of the housing to an external environment.

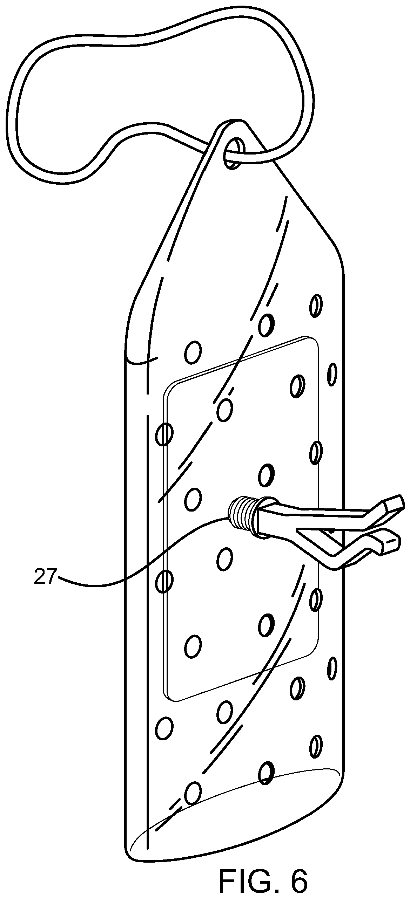

FIG. 6 shows an arrangement in which a clip is held by a screw 27. This may be compared to the interference fit shown in FIG. 3, in which a clip is inserted and then twisted 90 degrees to be secured.

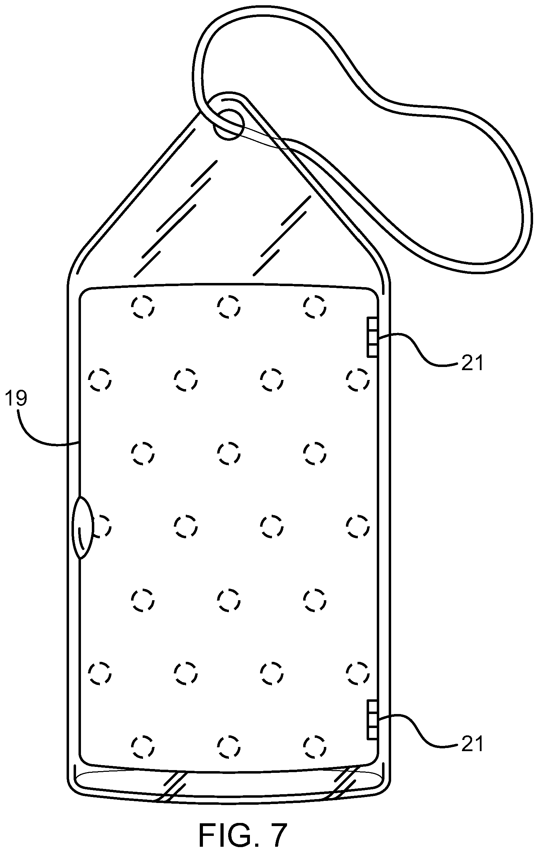

FIG. 7 shows an embodiment of the invention in which holes for fragrance are covered by a hard cover **19** movable by hinges **21** to allow the user to vary the flow of fragrance.

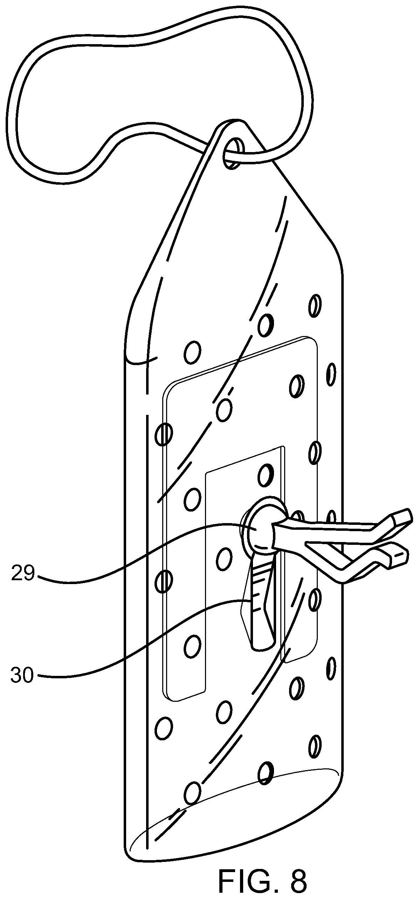

FIG. 8 shows an embodiment in which a clip is held by a ball and socket attachment. This figure also shows a recess 30 into which the clip may be placed when not in use.

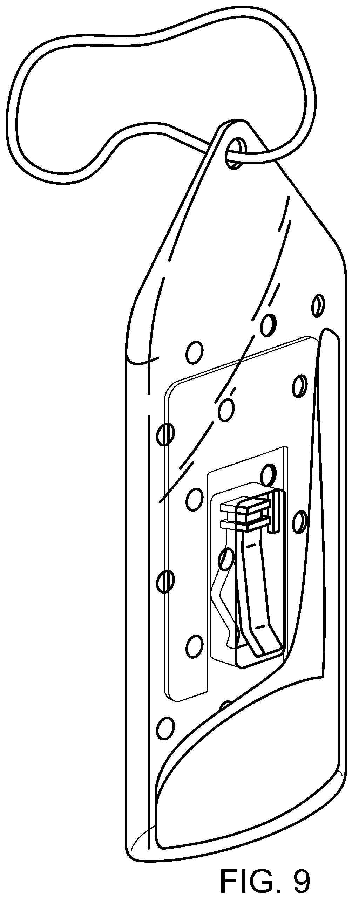

FIG. 9 shows a fragrance device according to one embodiment of the invention, in which a housing **1** includes a label 3. An attaching device, in this case a clip 5 is also shown. The clip is within a recess, and the recess is, in turn, covered by a label.

While the invention will be described in connection with certain preferred embodiments, there is no intent to limit it to those embodiments. On the contrary, the intent is to cover all alternatives, modifications and equivalents as included within the scope of the invention as defined by the appended claims.

### DETAILED DESCRIPTION OF THE INVENTION

Embodiments of the invention provide a fragrance distribution device comprising a housing. The housing includes an aperture into which a hook, clip, adhesive peg, or other attaching device may be secured. Typically the aperture is covered by a label, decal, or other cover until its use is desired. The housing encloses a polymer or thermoplastic fragrance earner. Typically the housing is plastic, though it may be other materials as desired, and the material of the housing is not critical.

The attaching device is removably secured to the housing. By "removably secured" it is meant that the attaching device may be repeatedly removed from and replaced in the housing without damaging the housing or the attaching device. This is done by having the attaching device able to be freely inserted into a slot, and then twisted to be secured.

In typical embodiments the housing is attached to a loop or other device that will allow the device to be placed over a knob, mirror, or other protrusion. This kind of temporary placement allows the device to passively release fragrance without attachment to a vent or fan.

The aperture(s) in the fragrance device may have any of a number of shapes and configurations. For example, it may be a horizontal, vertical, or diagonal slot. It may be a circular hole. The hole may be threaded or unthreaded. The aperture may be centered in the housing or off-center.

The housing may be any shape. Typically the housing includes vents on one or more of the side, front, or reverse of the housing, allowing the fragrance to escape the housing. The vents may be any shape but are typically holes or slots. In some embodiments the vents are on the same surf ace as the aperture.

In some embodiments the aperture is covered when not in use. This may be done by a decal, sticker, or other adhesive. In other embodiments the aperture may be closed by a more substantial cover, for example a hard plastic cover. This cover may be attached, for example by hinges or by an interference fit. The cover may be able to be replaced after it has been removed, or its removal may be permanent. In some embodiments the covering over the aperture is punctured rather than removed.

Removal of the cover may reveal one or more vent openings in the surface of the housing. In some embodiments a user may elect to reveal less than all of the vent openings when removing the cover.

### Polymer Carriers

Embodiments of the invention include the fragrances in a polymer or thermoplastic earner. One preferred polymeric carrier is ethylene vinyl acetate (EVA). EVA is a copolymer of ethylene and vinyl acetate. The EVA has no odor by its nature, however, it can adsorb or otherwise be permeated with a fragrance. EVA approaches elastomeric materials in softness and flexibility, yet can be processed like thermoplastics.

EV A used in the invention may have a molecular weight in the range of, for example, 10,000 Daltons to 100,000 Daltons, more preferably 22,000 to 87,000 Daltons. Fragrance may be introduced into the polymer at weight percents varying from 10 to 90%, from 20 to 80% from 30 to 70%, from 30 to 60%, and from 30 to 50%. In further embodiments, fragrance is introduced into the polymer at a weight percent of about 1 %, about 10%, about 20%, about 30%, about 40%, about 50%, about 60%, about 70%, about 80%, about 90%, or about 95%.

Other elastomeric or thermoplastic carriers or combinations thereof may be used as carriers, so long as they will release fragrance upon heating. Other suitable polymeric materials share the beneficial properties of EV A and may be substituted for use in embodiments of the invention. These include, for example, but are not limited to, high density polyethylene, low density polyethylene, polystyrene, acrylic polymers, polycarbonates, polyurethanes, nylons, and mixtures and copolymers of the foregoing.

Gellants may also be used as a fragrance support. For example, an IFO gel (for example, a polyamide gel) is one suitable example of a gellant that may be used in embodiments of the invention to replace all or part of the polymer component.

### Fragrances

One or more fragrances or odor neutralizers may be used in embodiments of the invention. If addition of fragrance is desired, suitable fragrances may be selected from those compiled by the U.S. Food and Drug Administration in Title 21 of the Code of Federal Regulations, Sections 172.510 and 172.515. Fragrance components selected from benzaldehydes, phenols, cinnamic aldehydes and esters, octadienes, dienes, cyclohexadienes, and terpenes may be used in the invention. Fragrance oils are also suitable for use alone or in combination with other fragrance chemicals. Suitable fragrance oils are, for example spice oil, flower oil, and fruit oil. Although fragrances are featured in many embodiments as reported here, other things deliverable by air including odor neutralizers and insect repellents may also be used.

Other suitable fragrances include but are not limited to benzyl alcohol, ethyl maltol, furaneol, 1-hexanol, cis-3-hexen-1-ol, menthol, benzaldehyde, hexanal, cinnamaldehyde, citral, cis-3-hexenal, furfural, neral, vanillin, ethyl acetate, ethyl butanoate, ethyl decanoate, ethyl hexanoate, ethyl octanoate, hexyl acetate, isoamyl acetate, methyl butanoate, methyl salicylate, pentyl butanoate, pentyl pentanoate, sotolon, strawberry aldehyde, fructone, anethole, anisole, eugenol, dihydrojasmone, 2-acetyl-1-pyrroline, 6-acetyl-2,3,4,5- tetrahydropyridine, gamma-decalactone, gamma-nonalactone, delta-octalactone, jasmine lactone, massoia lactone, camphor, citronellol, linalool, nerol, nerolidol,alpha-terpineol, thujone, and thymol.

In further embodiments of the invention, fragrances and/or odor neutralizers are mixed with one or more hindered amines. The hindered amines useful in the instant invention are well known in the art and are described in detail in U.S. Pat. No. 6,221,115. Examples of the hindered amines are: 1-(2-hydroxy-2-ethylpropoxy)-4-octadecanoyloxy-2,2,6,6-tetramethylpiperi- dine; 1-(2-hydroxy-2-methylpropoxy)-4-hydroxy-2,2,6,6-tetramethylpiperidin-e; bis(1-octyloxy-2,2,6,6-tetramethylpiperidin-4-yl) sebacate; bis(1-cyclohexyloxy-2,2,6,6-tetramethylpiperidin-4-yl) sebacate; 1-cyclohexyloxy-2,2,6,6-tetramethyl-4-octadecylaminopiperidine; 2,4-bis[(lcyclohexyloxy-2,2,6,6-tetramethylpiperidin-4-yl)butylamino]-6--(2-hydroxyeth ylamino-striazine; bis(1-cyclohexyloxy-2,2,6,6-tetramethylpiperidin-4-yl)adipate; 1-(2-hydroxy-2-methylpropoxy)-4-oxo-2,2,6,6-tetramethylpiperidine; bis(1-(2-hydroxy-2-methylpropoxy)-2,2,6,6-tetramethylpiperidin-4-yl)sebacate; bis(1-(2-hydroxy-2-methylpropoxy)-2,2,6,6- tetramethylpiperidin--4-yl)adipate; bis(1-(2-hydroxy-2-methylpropoxy)-2,2,6,6-tetramethylpiperidin-4-yl)succinate; bis(1-(2-hydroxy-2methylpropoxy)-2,2,6,6-tetramethylpiperidin-4-yl)glutarate; and 2,4-bis{N-[1-(2-hydroxy-2-methylpropoxy)-2,2,6,6-tetramethylpiperidin-4-y-1]-N-butylamino}-6-(2-hydroxyethylamino)-s-triazine)1-methoxy-4-hydroxy-2,2,6,6-tetramethylpiperidine; 1-methoxy-4-hydroxy-2,2,6,6-tetramethylpiperidine; loctyloxy-4-hydroxy-2,2,6,6-tetramethylpiperidine; 1-cyclohexyloxy-4-hydroxy-2,2,6,6-tetramethylpiperidine; 1-methoxy-4-oxo-2,2,6,6-tetramethylpiperidine; 1-octyloxy-4-oxo-2,2,6,6-tetramethylpiperidine; and 1-cyclohexyloxy-4-oxo-2,2,6,6-tetramethylpiperidine, or a mixture thereof.

In yet further embodiments of the invention, fragrances and/or odor neutralizers include one or more antioxidants. Antioxidants used in embodiments of the invention may be, for example, tertiary butylhydroquinone, n-octadecyl 3,5-di-tert-butyl-4-hydroxyhydrocinnamate, butylated hydroxyanisole, phenol bisphosphite, butylated hydroxytoluene, and phosphite compounds. An effective amount of antioxidant in the instant composition is 0.015% to 2.5% by weight of the EV A or other polymer, preferably 0.1 to 0.75% by weight and most preferably 0.2 to 0.5% by weight. In preferred embodiments of the invention, high concentrations of antioxidants are mixed with fragrance prior to addition of the fragrance/antioxidant mixture to any other components of the mixture.

Still further embodiments of the invention contemplate inclusion of the fragrance and/or odor neutralizer in a diluent prior to incorporation into a polymeric carrier. A diluent is organic, for example: triethyl citrate; di-isopropropyl adipate; di-octyl adipate; isopropyl myristate; isopropyl palmitate; butyl stearate; benzyl alcohol; benzyl benzoate; and diethyl pthalate. The quantity of diluent preferred is the quantity necessary for dissolving the fragrance or the antioxidant.

In one preferred embodiment, a selected fragrance and/or an odor neutralizer (with or without the other additives reported above) is embedded in and/or adsorbed on the polymer. Further information regarding creation of a fragrance/antioxidant/diluent mixture may be found in U.S. Patent No. 7,220,288.

Various other additives such as color additives may be added in different embodiments depending on desired characteristics of a particular fragrance device.

Plasticizers may also be added to polymeric materials that are used in embodiments of the invention. These may include, for example, diethyl phthalate and tri-acetic acid ester of glycerin.

### Examples

The following examples are given to help those skilled in the art appreciate the invention. They should not be construed to limit the scope of the claims.

### Example 1

Example 1 describes the construction of a polymer support containing a fragrance. An amount of fragrance that is 33.4% by weight of the anticipated final formula amount is weighed and loaded into a vessel. Into the fragrance is added antioxidant and hindered amine at 0.7% and 0.7% by weight of the anticipated final formula amount, respectively. An amount of 0.2% of a color solution is added. This is mixed until dissolution.

The fragrance solution is transferred to a 5 gallon drum. EVA in the amount of 65% by weight of the anticipated final formula amount is added as solid beads. The drum is sealed and rotated until the fragrance mixture is completely absorbed into the EVA.

The beads are loaded into an injection molding machine. It is preferred that prior to injection molding the beads be no larger than 325 mesh. A fragrance support is created in the desired shape through the injection molding process. The finished fragrance support may be packaged for sale as a refill item or may be placed within a housing.

Once the polymer support has been created, it is inserted into a housing. The housing is, for example, of a clamshell design. Opposite sides of the housing may be held by an interference fit, by an adhesive, by melting, or by other methods as appreciated by those in the art.

## Claims

1. A fragrance device comprising:
a housing (1) comprising a hollow interior, at least one vent (9) placing the interior in communication with an external environment, and a recess (30) in an external wall of the housing (1);
a polymer support (7) within the hollow interior, said support (7) including at least one of a fragrance, an insect repellent, and an odor neutralizer; and
an attachment device, said attachment device capable of being disposed within the recess (30) and removed from the recess (30) while being secured to the housing (1), said attachment device being removably secured to the housing (1) by being freely inserted into a slot formed into the recess (30).

2. The fragrance device of claim 1, wherein the at least one vent (9) is covered by a decal applied to the external wall.

3. The fragrance device of claim 1, wherein the attachment device, when disposed within the recess (30), is covered by at least one member of the group consisting of a decal and a hinged cover.

4. The fragrance device of claim 1, wherein the attachment device is selected from the group consisting of a clip (5), an adhesive peg (23), and a hook (25).

5. The fragrance device of claim 1, wherein the attachment device is secured to the housing by a member of the group consisting of an interference fit and a ball and socket.

6. The fragrance device of claim 1, wherein the polymer support (7) is ethylene vinyl acetate.

7. The fragrance device of claim 1, wherein the fragrance includes at least one member of the group consisting of benzaldehydes, phenols, cinnamic aldehydes and esters, octadienes, dienes, cyclohexadienes, and terpenes.

## Patentansprüche

1. Duftstoffvorrichtung, die Folgendes umfasst:
ein Gehäuse (1), das ein hohles Inneres, mindestens eine Entlüftung (9), die das Innere in Kommunikation mit einer externen Umgebung bringt, und eine Vertiefung (30) in einer externen Wand des Gehäuses (1) umfasst;
einen Polymerträger (7) innerhalb des hohlen Inneren, wobei der Träger (7) mindestens eines einschließt von einem Duftstoff, einem Insektenabwehrmittel und einem Geruchsneutralisator; und
eine Anbringungsvorrichtung, wobei die Anbringungsvorrichtung dazu in der Lage ist, innerhalb der Vertiefung (30) angeordnet und aus der Vertiefung (30) entfernt zu werden, während sie an dem Gehäuse (1) angebracht ist, wobei die Anbringungsvorrichtung beweglich an dem Gehäuse (1) angebracht ist, indem sie frei in einen Schlitz eingeführt ist, der in der Vertiefung (30) ausgebildet ist.

2. Duftstoffvorrichtung nach Anspruch 1, wobei die mindestens eine Entlüftung (9) von einem Aufkleber abgedeckt ist, der auf die externe Wand aufgebracht ist.

3. Duftstoffvorrichtung nach Anspruch 1, wobei die Anbringungsvorrichtung, wenn sie innerhalb der Vertiefung (30) angeordnet ist, von mindestens einem Element der Gruppe bestehend aus einem Aufkleber und einer Abdeckklappe bedeckt ist.

4. Duftstoffvorrichtung nach Anspruch 1, wobei die Anbringungsvorrichtung ausgewählt ist aus der Gruppe bestehend aus einem Clip (5), einem Klebehaken (23) und einem Haken (25).

5. Duftstoffvorrichtung nach Anspruch 1, wobei die Anbringungsvorrichtung durch ein Element der Gruppe bestehend aus einer Presspassung und einer Kugelgelenkverbindung an dem Gehäuse angebracht ist.

6. Duftstoffvorrichtung nach Anspruch 1, wobei der Polymerträger (7) Ethylenvinylacetat ist.

7. Duftstoffvorrichtung nach Anspruch 1, wobei der Duftstoff mindestens ein Element der Gruppe bestehend aus Benzaldehyden, Phenolen, Zimtaldehyden und Zimtsäureestern, Octadienen, Dienen, Cyclohexadienen und Terpen einschließt.

## Revendications

1. Dispositif de parfum comprenant :
un logement (1) comprenant un intérieur creux, au moins un évent (9) plaçant l'intérieur en communication avec un environnement externe, et un évidement (30) dans une paroi externe du logement (1) :
un support de polymère (7) à l'intérieur de l'intérieur creux, ledit support (7) incluant au moins l'un d'un parfum, d'un insectifuge et d'un neutralisant d'odeur ; et
un dispositif de fixation, ledit dispositif de fixation étant capable d'être disposé à l'intérieur de l'évidement (30) et retiré de l'évidement (30) tout en étant fixé au logement (1), ledit dispositif de fixation étant fixé de manière amovible au logement (1) en étant librement inséré dans une fente formée dans l'évidement (30).

2. Dispositif de parfum selon la revendication 1, dans lequel l'au moins un évent (9) est recouvert par une étiquette appliquée sur la paroi externe.

3. Dispositif de parfum selon la revendication 1, dans lequel le dispositif de fixation, lorsqu'il est disposé à l'intérieur de l'évidement (30), est recouvert par au moins un élément du groupe constitué d'une étiquette et d'un couvercle rabattable.

4. Dispositif de parfum selon la revendication 1, dans lequel le dispositif de fixation est choisi dans le groupe constitué d'une attache (5), d'une cheville adhésive (23) et d'un crochet (25).

5. Dispositif de parfum selon la revendication 1, dans lequel le dispositif de fixation est fixé sur le logement par un élément du groupe constitué d'un ajustement serré et d'une rotule.

6. Dispositif de parfum selon la revendication 1, dans lequel le support de polymère (7) est l'acétate de vinyle-éthylène.

7. Dispositif de parfum selon la revendication 1, dans lequel le parfum inclut au moins un élément du groupe constitué des benzaldéhydes, des phénols, des aldéhydes et des esters cinnamiques, des octadiènes, des diènes, des cyclohexadiènes et des terpènes.
